# EUROPEAN PATENT SPECIFICATION

(11) **EP 0 305 497 B1**
(45) Date of publication and mention of the grant of the patent: **03.11.1993**
(21) Application number: 88903100.1
(22) Date of filing: 15.03.1988
(51) Int. Cl.: A61B 10/00, G01N 33/53

(54) **FERTILITY PREDICTION BY USE OF CLOMIPHENE CHALLENGE TEST**
FRUCHTBARKEITSVORHERSAGE DURCH VERWENDUNG EINES CLOMIPHEN-KONTROLLSATZES
PREVISION DE LA FERTILITE PAR UTILISATION D'UN TEST DE CONFRONTATION AU CLOMIFENE

(30) Priority: 17.03.1987 US 26879
(43) Date of publication of application: 08.03.1989
(73) Proprietor: NAVOT, Daniel, Tenafly, NJ 07670 (US)
(72) Inventor: NAVOT, Daniel, Tenafly, NJ 07670 (US)
(74) Representative: Jones, Helen Marjorie Meredith
(86) International application number: US8800813
(87) International publication number: WO8806863

(56) References cited:
- FR-A- 2 146 865
- US-A- 4 278 668
- US-A- 4 339 434
- US-A- 4 514 505
- "OVUKIT self test for ovulation prediction", 1988, Monoclonal Antibodies, Inc., Sunnyvale, CA, US;
- Endocrinology, vol. 84, no. 4, issued April 1969 (William and Wilkins, Baltimore, MD 21202), H. Baier et al, "Effect of Clomiphene Upon Plasma FSH Activity ...", pages 946-949
- American Journal of Obstetrics and Gynecology, vol. 107, no. 8, issued 15 August 1970 (C.V. Mosby Co., St. Louis, MO 63146), A.V. Schally et al, "Alteration of LH and FSH release in rats treated with clomiphene or its isomers", pages 1156-1167
- Chemical Abstracts, vol. 76, no. 23, issued 5 June 1972 (Chemical Abstracts Service, Columbus OH 43210) page 60, abstract No. 135965h, G. Giustina et al, "Clomiphene induced modifications of plasma LH measured by radioimmunoassay"
- Chemical Abstracts, vol. 105, no. 15, issued 13 October 1986 (Chemical Abstracts Service, Columbus OH 43210), page 98, abstract no. 127655j, N. Terakawa et al, "A possible role of clomiphene citrate in the control of preovulatory LH surge ..."

## Description

### FIELD OF THE INVENTION

The present invention generally relates to methods for predicting female fertility. More particularly this invention relates to methods of predicting human female fertility based upon relates to methods of predicting human female fertility based upon chemical tests for detecting the levels of follicle stimulating hormone or luteinizing hormone in the female's urine or blood.

### BACKGROUND OF THE INVENTION

The fertility processes which occur in females, especially in human females, are highly complex, and continuing efforts are being made in this area to more fully understand these processes so as to facilitate the development of the improved techniques for predicting and treating fertility problems as well as for providing more effective and reliable birth control methods. A number of drugs have been employed to treat infertility. Clomiphene citrate is one such drug. However, in the fertility problem area, there still is an acute need for improved methods for studying, as opposed to treating, women who wish to conceive but are unable to do so. For example, a woman seeking a career outside the home may consider postponing childbearing until her thirties, and a prognosticator of fecundity would be of special value to her. It might guide her decisions regarding pregnancy attempts and contraception. It also would aid the physician in clinical decisions regarding infertility workup or therapy. Moreover, this need extends to women experiencing other difficulties such as menstrual cycle variations and other problems associated with the female child bearing organs. However, for the apparently healthy, regularly menstruating woman, age is presently the only widely accepted parameter of fertility potential. Demographic studies indicate that fertility peaks during the early twenties, decreases noticeably after 30 years of age and markedly after 35 years. However, because of individual variations, age by itself is an inaccurate parameter, and arbitrary limits based on age alone can influence treatment decisions in a number of patients.

In the birth control area, there is also an acute need for improved birth control methods which are not only effective in preventing conception but also have a reduced incidence of adverse physical side effects. There are a wide variety of birth control methods available, but these are often unacceptable due to the fact that they are unreliable (i.e., prevention of conception is not assured), or for medical reasons, or on religious grounds. One example of a birth control method which is unreliable is the so-called "rhythm" method, which is based on the fact that the woman is not fertile, i.e., ovulation has not occurred or is not about to occur, during a certain period in her menstrual cycle. The major disadvantage associated with this method is that, while the time period from when ovulation naturally occurs to the next succeeding menstrual period is essentially fixed in all women, the time period between the beginning of a menstrual period and the next ovulation can vary considerably depending on the particular woman concerned. It is during the time period between the beginning of a menstrual period and the onset of the next succeeding ovulation that sexual intercourse can occur without conception occurring since during this period the female ovum has not yet been produced by the woman. However, significant risks do exist with the "rhythm" method since, even if the woman has not ovulated at the time of sexual intercourse, ovulation occurring one or two days thereafter can result in conception since the life span of male sperm in the vaginal can be as long as one to two days, and sometimes longer.

Some methods do exist for determining when a woman is about to ovulate but these are inconvenient and difficult to interpret. One such method requires the woman to take her temperature every morning and to plot this on a graph. From the shape of the graph, it is possible to see when ovulation has actually occurred, but the major problems associated with this method is that fluctuations in body temperature can occur for many reasons other than the ovulation process.

It is also well known to this art that a surge in the level of human luteinizing hormone (hLH or HL) takes place about 34 hours prior to ovulation. It takes place according to considerations which can begin by first noting that the normal human menstrual cycle is divided into the follicular phase, ovulation, and the luteal phase. Normal hormonal control during the follicular phase produces maturation of the primary ovarian follicle. Human luteinizing hormone and human follicle stimulating hormone (hFSH or FSH) are known to have a sensitizing effect upon the primary ovarian follicle. These hormones act in concert to stimulate estrogen synthesis. It is also known that estrogen and hFSH achieve their sensitizing effects by inducing expression of gonadotropin receptors in preparation for ovulation. Estrogen in turn acts on the hypothalamus to control pituitary secretion of gonadotropins. Estrogen reaches a peak one to two days prior to ovulation. This peak in turn induces a positive feedback response in the anterior pituitary to hypothalamic gonadotropin releasing hormone. During this peak period, estrogen levels decrease while progesterone levels start to increase and thereby stimulate the release of high levels of hLH. This surge in the hLH level reaches peaks which are usually two to three times the preceding basal concentrations. This hLH surge in turn induces a rupture of the primary ovarian follicle and the resulting release of a mature oocyte. This phenomenon is generally referred to as "ovulation". Thereafter, lLH promotes luteinization and formation of the corpus luteum. This is followed by a decrease in the hLH level to baseline levels within two days in response to the peaking progesterone levels which serve to initiate the luteal phase which lasts about 14 days. In the absence of fertilization of the oocyte, a new follicle begins the selection procedure for maturation in the next menstrual cycle.

The methods for detecting the hLH surge have also improved in recent times. Before the development of immunoassays, analyses of hLH in urine was obtained by bioassay techniques. However, the clinical utility of these methods was limited; they had relatively low sensitivity and frequently required that urine extracts be tested. In the mid 1960's however, the introduction of radioimmunoassay for hLH provided a new tool for quantitating low levels hLH in urine or serum. The later introduction of enzyme immunoassays for hLH offered the further advantage of good sensitivity without the use of radioisotopes. Nonetheless, there still exists a need for improved methods for evaluating fertility in woman so that fertility problems can be alleviated or so that appropriate birth control methods can be selected. Moreover, the diagnosis of infertility problems is still hampered by the fact that prediction of ovulation does not in and of itself give any information as to whether or not the oocyte which is about to be released is one that is fecund.

Thus even though it is well known that the detection of the hLH surge can act as an important tool in the detection of ovulation since the onset of the hLH surge precedes ovulation by about 34 hours; and even though it is also known that peak hLH levels occur several hours later in urine than in serum with the onset of the surge in urine being about 30 hours before ovulation, it has not been heretofore appreciated that differences in hLH and/or hFSH levels before and after ovulation could be used in a diagnostic test aimed at determining whether or not the female is releasing fecund oocytes.

### SUMMARY OF THE INVENTION

The disclosed clomiphene challenge test can be utilized to prospectively assess future fertility potential in women, especially in women greater than 35 years of age with unexplained infertility. The test has four basic steps. A baseline hormone (most preferably FSH and/or LH) level is established; clomiphene is administered; a response hormone level is established; and the response level of the subject hormone is compared to its baseline level. The baseline period hormone level is preferably established on days 2-3. The response period level is preferably established on days 9-11. The human follicle-stimulating hormone (hFSH) and/or, human luteinizing hormone (hLH) levels are measured before and after administration of from about 50 mg to about 150 mg/per day of clomiphene between the baseline period and the response period. The clomiphene can be any clomiphene salt in any pharmaceutically acceptable carrier. However, in the most preferred embodiment of this invention, clomiphene is administered in the form of clomiphene citrate tablets. It can be administered between days 2 and day 9 of the menstrual cycle. However, daily administration on days 5, 6, 7, 8 and 9 is a highly preferred administration procedure and dosages of about 100 mg/day of clomiphene citrate, in the form of clomiphene tablets, administered at the same time daily, is the most preferred administration procedure.

The disclosed clomiphene challenge test can be performed upon urine and/or blood samples. However, for reasons of simplicity, urine is the preferred sample source. Urinary or serum FSH or LH each can be measured in a number of known semiquantitative fashions. The tests can also be based on combined FSH and LH levels. Test for FSH alone are, however, a preferred method of conducting the test. Again for reasons of simplicity, colorimetric tests are preferred to other test procedures which could include other, less preferred test procedures such as, for example, radioimmunoassay or pathological tests. Most preferably, the colorimetric tests employed are those based upon color reactions of monoclonal antibody-based, enzyme immunoassay tests. Such colorimetric test results can be interpreted by the woman with the aid of color charts which are - preferably made a part of a test kit.

For example, semiquantitative tests of human luteinizing hormone (hLH) in urine can be made by comparing certain color reactions which can be associated with a monoclonal antibody/hLH reaction. One such color reaction is described in a publication by Monoclonal Antibodies, Inc., entitled, "Ovustick™ Urine hLH Kit" (1984).

Generally, it discloses that human luteinizing hormone is a glycoprotein hormone comprised of two noncovalently bound polypeptide subunits, designated alpha and beta units, having carbohydrate side chains. The amino acid sequence of alpha-hLH is essentially the same as that of human folicle-stimulating hormone. It has been established that the beta subunit of hLH is responsible for the biological and immunochemical specificity of this particular hormone.

Hence an hLH test kit normally used to chart ovulation could also be used as the test method to practice the herein disclosed clomiphene challenge test. Preferably it will be in the form of a visual determinable, enzyme immunoassay that incorporates the advantages of monoclonal antibody technology. Such antibodies offer the great advantage of controlled specificity and affinity. They will provide consistent assay performance since they enter into a specifically determined color reaction whose results can be compared by simple color comparison charts. Hence, tests utilizing monoclonal in semiquantitative, two-site, enzyme linked immunospecific assays are most preferred in the practice of their invention. The specificity of these tests follows from the fact that these hormones in a urine specimen can be sandwiched between the alpha-subunit-specific antibody, which has been immobilized on a plastic dipstick, and a beta specific antibody, such as beta hLH, which has been linked to the enzyme alkaline phosphatase. After unbound enzyme conjugate is removed by washing, the stick can be incubated in a substrate solution. The substrate reacts with the enzyme and deposits a blue end product on the reactive end of the stick. By way of example then, a semiquantitative analysis of hLH in urine can be made by comparing the intensity of the color on each stick with the color on the sticks incubated in, for example, concurrently run 0 mIU/ml, 20 mIU/ml and 40 mIU/ml calibrators. Color changes corresponding to response levels above one standard deviation, and more preferably those indicating response levels more than two standard deviations, above the base level can be considered as an indication of a low level of fecundity.

### DESCRIPTION OF PREFERRED EMBODIMENTS

### Populations and Methods

The herein disclosed clomiphene citrate challenge tests were utilized to prospectively assess future fertility potential in women greater than 35 years of age with unexplained infertility. Baseline (day 2-3) and response levels (day 9-11) of follicle stimulating hormone (FSH), luteinizing hormone (LH), and 17-beta estradiol (E₂) were measured before and after administration of 100 mg clomiphene on days 5-9 of the menstrual cycle. Sixteen women had an exaggerated FSH response of over 26 mIU/ml or more (2 standard deviations (SD) above levels found in normal controls), which was considered a diminished ovarian reserve (DOR). Mean baseline FSH in 16 women in the DOR group was 13.5 plus or minus 5 SD mIU/ml with a response level of 38.9 plus or minus 13.8 mIU/ml, while the baseline in 30 women with adequate ovarian reserve (AOR) was 9 plus or minus 4.2 mIU/ml with a response level of 11.5 plus or menus 4.9 mIU/ml. There was a highly significant difference (p,0.0001) between FSH response levels of the two groups and between response to baseline levels in the DOR group. LH response was consistently greater than FSH response in the AOR group, while consistently less than FSH in the DOR group (P<0.001). One of 16 patients in the DOR group conceived; 14 of 30 (47%) conceived in the AOR group (p,0.005). These tests indicate that despite apparently "normal" ovulatory cycles, the DOR group has compromised follicular apparatus. Disparity between normal E₂ secretory capacity of the granulosa and diminished capacity to secrete inhibin could explain the inappropriately high FSH levels in response to the clomiphene citrate challenge test. Hence it would appear that this clomiphene challenge test can reliably predict fecundity irrespective of age. It can provide a prospective test of the reserves of the gonadal hypothalamic axis as an indicator of female fecundity.

The basic study to establish this invention was conducted upon fifty-one patients seeking counsel for infertility between June, 1983, and January, 1986. Criteria for inclusion were age of 35 years or above, regular menstrual periods (23-35 days), normal seminal fluid analysis of the partner, and no history suggestive of mechanical causes of infertility. All patients were followed until conception or up to 3.5 years. Within one year, if no pregnancy ensued, infertility workup was completed or re-evaluated. Four patients were found to suffer from multiple pelvic adhesions, and one husband had repeatedly subfertile semen analyses. These five were excluded from the statistical analysis of pregnancy.

The ovarian challenge test was based on the administration of clomiphene citrate (CC), 100 mg/day for five consecutive days, starting on day 5 of the menstrual cycle. Peripheral venous blood was drawn during the early (days 2-3) and late (days 9-11) follicular phase. Hormone levels were measured by standard radioimmunoassay (RIA) for follicle stimulating hormone (FSH), luteinizing hormone (LH) (Amersham Radiochemical Center, Amersham, England, according to 2nd IRP human menopausal gonadotropin (HMG) reference standards), 17-B estradiol (E₂), progesterone and dehydroepianirosterone sulphate (DHEA-S) all by commercially available RIA kits. Hormone levels on cycle day 2 or 3 are referred to as the baseline hormonal profile, while days 9 to 11 are the preferred times to establish the response values.

An FSH value higher than 26 mIU/ml, measured on days 9-11, was defined as an abnormal response and was designated as the diminished ovarian reserve (DOR) group. Patients whose response was within the normal range were defined as the adequate ovarian reserve (AOR) group. 26 mIU/ml of FSH is more than two standard deviations above the mean value on days 9-11 as observed in 8 healthy volunteers aged 22-28 years, undergoing the same clomiphene challenge tests. The results of the clomiphene challenge tests were separately filed. Fertility promoting treatment was conducted irrespective of the clomiphene challenge test results or interpretation. Nearly all the women (90%) eventually has ovulation stimulation with gonadotropins. The one DOR patient who conceived did so during an IVF cycle subsequent to failures of human menopausal gonadotropin (hMG) treatment for 12 previous cycles. All values are expressed as mean plus or minus standard deviation (SD). Statistical analysis was performed employing student's T test or the Fischer exact test as

### Results

Of the 51 women, 18 had an abnormal response to the clomiphene challenge tests; 33 had FSH response levels within 2 SD of mean normal value as defined above. Table 1 details the age, duration of infertility, duration of follow-up, gonadotropin treatment, and outcome in the 51 patients. The two groups of patients did not differ in age, duration of infertility, treatment, or mean follow-up, (see Table 1). Sixteen of eighteen women in the DOR group (89%) and 31 of 33 in the AOR group were treated with hMG and human chorionic gonadotropin (hCG) for ovulation induction, performed according to individually adjusted schedules. During treatment and follow-up, only 1 of 18 women in the DOR group conceived (6.3%) while 14 of 33 (46.7%) conceived in the AOR group. The difference in conception rates is statistically significant (p<0.005). The results of these tests are summarized in Table I.

It should be noted that the baseline hormonal profiles differed only in respect to FSH levels. Although well within the normal range (13.5 plus or minus 5 mIU/ml), the mean FSH level was significantly higher (p<0.002) in the DOR group than in the AOR group. No other baseline values differed between the two groups. A highly significant increase in serum FSH levels was observed after clomiphene citrate administration in the DOR group (p<0.0001), while only moderate increase was detected in the AOR population (p=0.05). The difference between the two groups in response levels of FSH was highly significant (p<0.0001). In both groups a significant elevation in LH levels from baseline was documented, although LH levels on days 9-11 were significantly higher in the DOR group than in the AOR group (p<0.02).

In 15 of 18 (83%) in the DOR group, FSH levels more than doubled, while in the AOR group only 3 of 33 (9%) had FSH levels twice as high after clomiphene citrate administration. This difference is statistically significant (p<0.0001). In the DOR group a reversal of the ratio of the FSH/LH response was also noted in the same proportion of patients; in 15 of 18, FSH levels were higher than LH levels on days 9-11, while only 3 of 33 in the AOR group had higher FSH levels that LH levels (p<0.0001). There was an obvious rise in E₂ after clomiphene citrate was administered in both groups, and higher levels were attained in the normal response group. However, none of the values differed significantly, probably because of the wide range of E₂ values.

P levels were less than 1 mg/ml in all but one patient sampled on days 2-3 and all but two patients sampled on days 9-11. Mean mid-luteal P levels are suggestive of ovulation in both groups: 11.4 plus or minus 3.4 vs 18.3 plus or minus 3.4 mg/ml in the DOR and AOR groups respectively. However, peak luteal P levels were significantly higher in the AOR group (p<0.05), see Table 1.

Despite a definite trend of elevation in delta ₄A, T, and DHEA-S levels after clomiphene citrate challenge, no statistically significant differences were found between baseline and response levels, and no discrimination could be demonstrated on the basis of ovarian reserve. Prolactin levels were somewhat lower after the clomiphene citrate challenge test, but the differences were not statistically significant.

### Discussion

These results indicate that a diminished fertility potential can be detected through the utilization of a clomiphene citrate challenge test. This longitudinal study demonstrates that fecundity can be predicted on the basis of gonadotropin response to clomiphene citrate stimulation. The challenge test has a 93.5% sensitivity in recognizing the population with diminished fertility potential and a 47% specificity in identifying the fertile population. The specificity is apparently low; however, the 47% conception rate is remarkable in a relatively old group of apparently normal patients undergoing ovulation induction. It may be that older patients who have unexplained fertility have subtle ovulatory abnormalities. The favorable results of hMG/hCG augmentation of ovulation suggest that a hyperstimulated cycle may overcome those undefined abnormalities and can achieve pregnancy when the natural cycle have failed. The fact that all patients had normal baseline FSH and LH values and none had entered menopause during the follow-up period suggests that an inadequate ovarian reserve may be detected a considerable time before actual cessation of menses. During the early stages of perimenopause, there is a gradual rise in serum gonadotropins due to decreasing members of oocytes and follicles. The rise in FSH levels is earlier and more marked than LH levels. In contradistinction to the perimenopause, with clomiphene citrate administration during the reproductive span of life there is an early rise in pituitary gonadotropin levels which is more marked and sustained for LH than for FSH. In DOR patients, gonadotropin response to the clomiphene citrate challenge is reversed, being much more pronounced for FSH than LH. This peculiar response pattern of gonadotropins is similar to premenopausal FSH/LH ratio and may be attributed to a decrease in the ovarian elaboration of inhibin. Inhibin can specifically suppress plasma FSH levels, probably by reducing the release of FSH from adenohypophysis. Like steroidal hormones, inhibin is secreted by granulosa cells to secrete inhibin may also be related to oocyte quality. A reduced capacity to secrete inhibin could explain the specific overshoot in FSH levels after the clomiphene citrate challenge. If that capacity is also related to oocyte quality, it may explain the severely compromised chances to conceive. Thus, a clomiphene citrate challenge may unmask incipient failure in an - apparently normal follicular apparatus.

The increase in T and delta-₄A following clomiphene citrate treatment is an established phenomenon. This increase in steroidogenesis is partly accounted for by the increase in LH levels which accelerate ovarian steroidogenesis. A direct action of clomiphene citrate on ovarian 3B-ol dehydrogenasedelta-₅ isomerase is an additional route through which generation of delta-₄A may increase. The elevated androgen levels during clomiphene citrate levels on days 9-11 were similar for the AOR and DOR groups; but this could not account for the significant difference in conception rates between the groups.

Hence this study shows that the herein disclosed clomiphene citrate challenge can be used as a stress test for the reserve of the ovarian-follicular apparatus. Therefore, it will prove helpful in clinical decisions regarding contraception, type and timing of profertility treatment, prognostication, and-above-all- knowing when to abandon further efforts in an already over treated infertile woman whose failure to conceive remains unexplained.

From the above discussion, those skilled in this art will appreciate that although the application of clomiphene citrate for treatment of infertility and for testing the integrity of the hypothalamic-pituitary-ovarian axis is well established, it has not been described in the context of evaluating fecundity in patients seeking treatment for infertility. Those skilled in the art will also appreciate that the herein disclosed clomiphene challenge test also can be modified without departing from the limits and spirit of this patent disclosure. At the very least, the form of the clomiphene (e.g., administration via various salt forms), the days of testing and the adminstration dosage can be varied somewhat in the practice of the hereinafter claimed invention.

## Claims

1. A method for predicting female fertility comprising:
administering clomiphene in the period of days 2 to 9 of the menstrual cycle; and
determining the response level of gonadotropin, in the period of days 9 to 11 of the menstrual cycle.

2. A method according to claim 1, wherein the response level is determined of follicle stimulating hormone as gonadotropin.

3. A method according to claim 1, wherein the response level is determined of luteinising hormone as gonadotropin.

4. A method according to claim 1, wherein the response levels are determined of follicle stimulating hormone and of luteinising hormone as gonadotropins.

5. A method according to claim 1, wherein the method additionally comprises determining a baseline level of gonadotropin in the period of days 2-3 of the menstrual cycle and comparing the baseline and response levels of gonadotropin.

6. A method according to claim 5, wherein the gonadotropin comprises follicle stimulating hormone.

7. A method according to claim 5 or claim 6, wherein the gonadotropin comprises luteinising hormone.

8. A method according to any preceding claim in which clomiphene is administered in the form of clomiphene citrate.

9. A method according to any preceding claim in which clomiphene is administered in the period of days 5 to 9 of the menstrual cycle, preferably on each day of that period.

10. A method according to any preceding claim in which clomiphene is administered in an amount in the range 50-150 mg/day, preferably in an amount of about 100 mg/day.

11. A method according to any preceding claim in which the level of gonadotropin in urine is determined.

12. A method according to any preceding claim in which the gonadotropin levels are determined colorimetrically using a test based on monoclonal antibodies to the gonadotropin, preferably comprising enzyme linked monoclonal antibodies.

13. A method according to claim 2, wherein a follicle stimulating hormone response level greater than two standard deviations above response levels found in healthy controls is predictive of infertility.

14. A method according to claim 2, wherein a follicle stimulating hormone response level greater than 26 mIU/ml is predictive of infertility.

15. A method according to claim 3, wherein a luteinising hormone response level significantly greater than response levels found in normal controls is predictive of infertility.

16. A method according to claim 4, wherein a follicle stimulating hormone response level greater than the luteinising hormone response level is predictive of infertility.

17. A method according to any of claims 13-16, wherein the level of gonadotropin in urine is determined.

18. A product comprising in combination clomiphene and assay means for determining the level of gonadotropin for use in a method for predicting female fertility comprising:
administering clomiphene in the period of days 2 to 9 of the menstrual cycle; and
determining the response level of follicle stimulating hormone as gonadotropin, in the period of days 9 to 11 of the menstrual cycle.

19. A product according to claim 18, wherein the assay means are for the determination of the response level of follicle stimulating hormone as gonadotropin.

20. A product according to claim 18, wherein the assay means are for the determination of the response level of luteinising hormone as gonadotropin.

21. A product according to claim 18, wherein the assay means are for the determination of the response levels of follicle stimulating hormone and of luteinising hormone as gonadotropins.

22. A product according to claim 18, wherein the product also comprises assay means for determining a baseline level of gonadotropin in the period of days 2-3 of the menstrual cycle.

23. A product according to claim 22, wherein both assay means are for the determination of the level of follicle stimulating hormone as gonadotropin.

24. A product according to claim 22, wherein both assay means are for the determination of the level of luteinising hormone as gonadotropin.

25. A product according to any of claims 18 to 24, wherein the or each assay means comprise monoclonal antibodies to the gonadotropin and a colorimetrically detectable component, and preferably comprise enzyme-linked monoclonal antibodies.

26. A product according to any of claims 18 to 25, wherein the or each assay means are for the determination of gonadotropin level in urine.

27. A product according to any of claims 18 to 26, wherein clomiphene is in the form of clomiphene citrate.

28. A product according to any of claims 18 to 27, wherein the clomiphene is in a form suitable for administration on each of days 5 to 9 of the menstrual cycle.

29. A product according to any of claims 18 to 28, wherein the clomiphene is in a form suitable for administration of a daily dose in the range 50 to 150 mg, preferably in a form suitable for administration of a daily dose of about 100 mg.

30. Use of clomiphene in a process of manufacturing a product for use in a method according to any of claims 1 to 17.

## Patentansprüche

1. Verfahren zur Voraussage der weiblichen Fruchtbarkeit, umfassend:
die Verabreichung von Clomiphen in der Zeitspanne der Tage 2 bis 9 des Menstruationszyklus; und
die Bestimmung des Gonadotropin-Ansprechspiegels in der Zeitspanne der Tage 9 bis 11 des Menstruationszyklus.

2. Verfahren nach Anspruch 1, in welchem der Ansprechspiegel von Follikel-stimulierendem Hormon als Gonadotropin bestimmt wird.

3. Verfahren nach Anspruch 1, in welchem der Ansprechspiegel von luteinisierendem Hormon als Gonadotropin bestimmt wird.

4. Verfahren nach Anspruch 1, in welchem die Ansprechspiegel von Follikel-stimulierendem Hormon und von luteinisierendem Hormon als Gonadotropinen bestimmt werden.

5. Verfahren nach Anspruch 1, in welchem das Verfahren zusätzlich die Bestimmung eines Gonadotropin-Grundlinienspiegels in der Zeitspanne der Tage 2-3 des Menstruationszyklus und den Vergleich der Gonadotropin-Grundlinien- und -Ansprechspiegel umfaßt.

6. Verfahren nach Anspruch 5, in welchem das Gonadotropin Follikel-stimulierendes Hormon umfaßt.

7. Verfahren nach Anspruch 5 oder Anspruch 6, in welchem das Gonadotropin luteinisierendes Hormon umfaßt.

8. Verfahren nach irgendeinem vorangehenden Anspruch, in welchem Clomiphen in Form von Clomiphencitrat verabreicht wird.

9. Verfahren nach irgendeinem vorangehenden Anspruch, in welchem Clomiphen in der Zeitspanne der Tage 5 bis 9 des Menstruationszyklus, vorzugsweise an jedem Tag dieser Zeitspanne, verabreicht wird.

10. Verfahren nach irgendeinem vorangehenden Anspruch, in welchem Clomiphen in einer Menge im Bereich von 50-150 mg/Tag, vorzugsweise in einer Menge von etwa 100 mg/Tag, verabreicht wird.

11. Verfahren nach irgendeinem vorangehenden Anspruch, in welchem der Gonadotropin-Spiegel im Urin bestimmt wird.

12. Verfahren nach irgendeinem vorangehenden Anspruch, in welchem die Gonadotropin-Spiegel colorimetrisch unter Verwendung eines auf monoklonalen Antikörpern gegen das Gonadotropin basierenden Tests bestimmt werden, vorzugsweise mit Enzym verknüpfte monoklonale Antikörper umfassend.

13. Verfahren nach Anspruch 2, in welchem ein Follikel-stimulierendes Hormon-Ansprechspiegel, der größer als zwei Standardabweichungen oberhalb der in gesunden Kontrollen gefundenen Ansprechspiegel ist, Unfruchtbarkeit voraussagt.

14. Verfahren nach Anspruch 2, in welchem ein Follikel-stimulierendes Hormon-Ansprechspiegel von größer als 26 mIU/ml Unfruchtbarkeit voraussagt.

15. Verfahren nach Anspruch 3, in welchem ein luteinisierendes Hormon-Ansprechspiegel, der merklich größer ist als die Ansprechspiegel, die in normalen Kontrollen gefunden wurden, Unfruchtbarkeit voraussagt.

16. Verfahren nach Anspruch 4, in welchem ein Follikel-stimulierendes Hormon-Ansprechspiegel, der größer als der lutenisierende Hormon-Ansprechspiegel ist, Unfruchtbarkeit voraussagt.

17. Verfahren nach irgendeinem der Ansprüche 13-16, in welchem der Gonadotropin-Spiegel im Urin bestimmt wird.

18. Produkt, umfassend in Kombination Clomiphen und Assay-Mittel zur Bestimmung des Gonadotropin-Spiegels zur Verwendung in einem Verfahren zur Voraussage der weiblichen Fruchtbarkeit, welches umfaßt:
Verabreichung von Clomiphen in der Zeitspanne der Tage 2 bis 9 des Menstruationszyklus; und
Bestimmung des Ansprechspiegels von Follikel-stimulierendem Hormon als Gonadotropin in der Zeitspanne der Tage 9 bis 11 des Menstruationszyklus.

19. Produkt nach Anspruch 18, in welchem die Assay-Mittel für die Bestimmung des Ansprechspiegels von Follikel-stimulierendem Hormon als Gonadotropin bestimmt sind.

20. Produkt nach Anspruch 18, in welchem die Assay-Mittel für die Bestimmung des Ansprechspiegels von luteinisierendem Hormon als Gonadotropin bestimmt sind.

21. Produkt nach Anspruch 18, in welchem die Assay-Mittel für die Bestimmung der Ansprechspiegel von Follikel-stimulierendem Hormon und luteinisierendem Hormon als Gonadotropinen bestimmt sind.

22. Produkt nach Anspruch 18, in welchem das Produkt auch Assay-Mittel zur Bestimmung eines Gonadotropin-Grundlinienspiegels in der Zeitspanne der Tage 2-3 des Menstruationszyklus umfaßt.

23. Produkt nach Anspruch 22, in welchem beide Assay-Mittel für die Bestimmung des Spiegels von Follikel-stimulierendem Hormon als Gonadotropin bestimmt sind.

24. Produkt nach Anspruch 22, in welchem beide Assay-Mittel für die Bestimmung des Spiegels von luteinisierendem Hormon als Gonadotropin bestimmt sind.

25. Produkt nach irgendeinem der Ansprüche 18 bis 24, in welchem das oder jedes Assay-Mittel monoklonale Antikörper gegen das Gonadotropin und eine colorimetrisch nachweisbare Komponente umfaßt und vorzugsweise mit Enzym verknüpfte monoklonale Antikörper umfaßt.

26. Produkt nach irgendeinem der Ansprüche 18 bis 25, in welchem das oder jedes Assay-Mittel für die Bestimmung von Gonadotropin-Spiegel im Urin bestimmt ist.

27. Produkt nach irgendeinem der Ansprüche 18 bis 26, in welchem Clomiphen in der Form von Clomiphencitrat vorliegt.

28. Produkt nach irgendeinem der Ansprüche 18 bis 27, in welchem das Clomiphen in einer für die Verabreichung an jedem der Tage 5 bis 9 des Menstruationszyklus geeigneten Form vorliegt.

29. Produkt nach irgendeinem der Ansprüche 18 bis 28, in welchem das Clomiphen in einer für die Verabreichung einer täglichen Dosis im Bereich von 50 bis 150 mg geeigneten Form vorliegt, vorzugsweise in einer Form, die für die Verabreichung einer täglichen Dosis von etwa 100 mg geeignet ist.

30. Verwendung von Clomiphen in einem Verfahren zur Herstellung eines Produkts zur Verwendung in einem Verfahren gemäß irgendeinem der Ansprüche 1 bis 17.

## Revendications

1. Méthode de prévision de la fertilité féminine comprenant :
l'administration de clomifène dans la période allant du jour 2 au jour 9 du cycle menstruel ; et
la détermination du niveau de réponse d'une gonadotrophine dans la période allant des jours 9 à 11 du cycle menstruel.

2. Méthode selon la revendication 1, dans laquelle le niveau de réponse déterminé est celui de l'hormone folliculostimulante en tant que gonadotrophine.

3. Méthode selon la revendication 1, dans laquelle le niveau de réponse déterminé est celui de l'hormone lutéinisante en tant que gonadotrophine.

4. Méthode selon la revendication 1, dans laquelle le niveau de réponse déterminé est celui de l'hormone folliculostimulante et de l'hormone lutéinisante en tant que gonadotrophines.

5. Méthode selon la revendication 1, comprenant en outre la détermination d'un niveau de base d'une gonadotrophine dans la période des jours 2-3 du cycle menstruel et la comparaison des niveaux de base et de réponse de la gonadotrophine.

6. Méthode selon la revendication 5, dans laquelle la gonadotrophine comprend l'hormone folliculostimulante.

7. Méthode selon la revendication 5 ou 6, dans laquelle la gonadotrophine comprend l'hormone lutéinisante.

8. Méthode selon une quelconque des revendications précédentes, dans laquelle le clomifène est administré sous la forme de citrate de clomifène.

9. Méthode selon une quelconque des revendications précédentes, dans laquelle le clomifène est administré dans la période des jours 5 à 9 du cycle menstruel, de préférence chaque jour de cette période.

10. Méthode selon une quelconque des revendications précédentes, dans laquelle le clomifène est administré selon une quantité allant de 50 à 150 mg/jour, de préférence selon une quantité d'environ 100 mg/jour.

11. Méthode selon une quelconque des revendications précédentes, dans laquelle on détermine le taux urinaire de gonadotrophine.

12. Méthode selon une quelconque des revendications précédentes, dans laquelle on détermine les taux urinaires de gonadotrophine par colorimétrie en utilisant un test fondé sur des anticorps monoclonaux pour la gonadotrophine, comprenant de préférence des anticorps monoclonaux liés à des enzymes.

13. Méthode selon la revendication 2, dans laquelle un niveau de réponse de l'hormone folliculostimulante supérieur de plus de deux écarts types aux niveaux de réponse observés chez les témoins sains est prédictif d'une stérilité.

14. Méthode selon la revendication 2, dans laquelle un niveau de réponse de l'hormone folliculostimulante supérieur à 26 mUI/ml est prédictif d'une stérilité.

15. Méthode selon la revendication 3, dans laquelle un niveau de réponse de l'hormone lutéinisante significativement supérieur aux niveaux de réponse des témoins normaux est prédictif d'une stérilité.

16. Méthode selon la revendication 4, dans laquelle un niveau de réponse de l'hormone folliculostimulante supérieur au niveau de réponse de l'hormone lutéinisante est prédictif d'une stérilité.

17. Méthode selon une quelconque des revendications 13 à 16, dans laquelle on détermine le taux urinaire de gonadotrophine.

18. Produit comprenant une association de clomifène et de moyens de dosage pour déterminer le taux de gonadotrophine pour utilisation dans une méthode de prévision de la fertilité féminine comprenant :
l'administration de clomifène dans la période allant du jour 2 au jour 9 du cycle menstruel ; et
la détermination du niveau de réponse de la gonadotrophine dans la période allant des jours 9 à 11 du cycle menstruel.

19. Produit selon la revendication 18, dans lequel les moyens de dosage sont ceux de l'évaluation du niveau de réponse de l'hormone folliculostimulante en tant que gonadotrophine.

20. Produit selon la revendication 18, dans lequel les moyens de dosage sont ceux de l'évaluation du niveau de réponse de l'hormone lutéinisante en tant que gonadotrophine.

21. Produit selon la revendication 18, dans lequel les moyens de dosage sont ceux de l'évaluation des niveaux de réponse de l'hormone folliculostimulante et de l'hormone lutéinisante en tant que gonadotrophines.

22. Produit selon la revendication 18, comprenant aussi des moyens de dosage pour déterminer un niveau de base de gonadotrophine dans la période des jours 2-3 du cycle menstruel.

23. Produit selon la revendication 22, dans lequel les moyens de dosage sont pour l'évaluation du niveau de réponse de l'hormone folliculostimulante en tant que gonadotrophine.

24. Produit selon la revendication 22, dans lequel les moyens de dosage sont pour l'évaluation du niveau de réponse de l'hormone lutéinisante en tant que gonadotrophine.

25. Produit selon une quelconque des revendications 18 à 24, dans lequel le ou chaque moyen de dosage comprend des anticorps monoclonaux pour la gonadotrophine et un composant décelable par colorimétrie et de préférence des anticorps monoclonaux liés à des enzymes.

26. Produit selon une quelconque des revendications 18 à 25, dans lequel le ou chaque moyen de dosage est destiné à la détermination du taux urinaire de gonadotrophine.

27. Produit selon une quelconque des revendications 18 à 26, dans lequel le clomifène est sous la forme de citrate de clomifène.

28. Produit selon une quelconque des revendications 18 à 27, dans lequel le clomifène est sous une forme adaptée à une administration chacun des jours 5 à 9 du cycle menstruel.

29. Produit selon une quelconque des revendications 18 à 28, dans lequel le clomifène est sous une forme adaptée à l'administration d'une dose quotidienne dans l'intervalle 50 à 150 mg, de préférence sous une forme adaptée à l'administration d'une dose quotidienne d'environ 100 mg.

30. Utilisation du clomifène dans un procédé de fabrication d'un produit à utiliser dans une méthode selon une quelconque des revendications 1 à 17.
